# EUROPEAN PATENT SPECIFICATION

(11) **EP 4 368 243 B1**
(45) Date of publication and mention of the grant of the patent: **25.06.2025**
(21) Application number: 23209043.1
(22) Date of filing: 10.11.2023
(51) Int. Cl.: A61N 1/36, A61B 5/00, A61B 5/055, A61B 6/12, A61B 8/08, A61B 5/06, A61B 8/00

(54) **SYSTEMS FOR PROGRAMMING A LEAD FOR THERAPEUTIC NEUROMODULATION**
SYSTEME ZUR PROGRAMMIERUNG EINER LEITUNG FÜR THERAPEUTISCHE NEUROMODULATION
SYSTÈMES DE PROGRAMMATION D'UNE DÉRIVATION POUR UNE NEUROMODULATION THÉRAPEUTIQUE

(30) Priority: 14.11.2022 US 202263425097 P; 07.11.2023 US 202318503215
(43) Date of publication of application: 15.05.2024
(73) Proprietor: Mazor Robotics Ltd., 3079830 Caesarea (IL)
(72) Inventor: Vigh, Rowena Ong, Louisville, 80027 (US); Nedrud, Joshua James, Minneapolis, 55432 (US); Cleland, Andrew Jay, Minneapolis, 55432 (US); Mahendra, Nikhil, Louisville, 80027 (US); Luo, Ma, Louisville, 80027 (US); Kearns, Erin L., Louisville, 80027 (US); Vlasov, Michael, 3079830 Caesarea (IL)
(74) Representative: Zimmermann & Partner Patentanwälte mbB

(56) References cited:
- US-A1- 2012 014 580
- US-A1- 2016 243 367
- US-B2- 10 265 531

## Description

### BACKGROUND

The present disclosure is directed to therapeutic neuromodulation and relates more particularly to programming a lead for therapeutic neuromodulation.

Closed-loop neuromodulation therapy may be carried out by sending an electrical signal generated by a pulse generator to a stimulation target (e.g., nerves, non-neuronal cells, etc.), which may provide a stimulating or blocking therapy to the stimulation target. In such closed-loop neuromodulation therapies, one or more signals resulting from the stimulating or blocking therapy may be recorded and the therapy may be adjusted based on the recorded signals.

US2016/243367 discloses a system for generating a lead parameter.

### BRIEF SUMMARY

The invention is defined by the appended claims. Systems and non-claimed methods for programming a lead are provided. A first image of an anatomical region depicting an anatomical element and at least a portion of an initial lead and an initial lead parameter associated with the initial lead are received. A second image of the anatomical region depicting at least a portion of the anatomical element and at least a portion of an implanted lead is also received. An implanted lead parameter may be generated for the implanted lead based on a combination of the initial lead parameter and a correlation of the first image with the second image.

Example aspects of the present disclosure include:
A system for generating a lead parameter according to at least one embodiment of the present disclosure comprises a pulse generator configured to generate an electrical signal; an implanted lead in communication with the pulse generator and configured to transmit the electrical signal to an anatomical element; s processor; and a memory storing data for processing by the processor, the data, when processed, causes the processor to: receive a first image of an anatomical region, wherein the first image depicts the anatomical element and at least a portion of an initial lead; receive an initial lead parameter associated with the initial lead; receive a second image of the anatomical region, wherein the second image depicts at least a portion of the anatomical element and at least a portion of the implanted lead, the second image being received at a different time than the first image; and generate an implanted lead parameter for the implanted lead based on a combination of the initial lead parameter and a correlation of the first image with the second image.

Any of the aspects herein, wherein the anatomical region comprises a target anatomical element, and wherein the target anatomical element comprises a vertebra and the anatomical element comprises a spinal cord.

Any of the aspects herein, wherein the memory stores further data for processing by the processor that, when processed, causes the processor to: process the first image to identify the target anatomical element and the initial lead; and process the second image to identify the target anatomical element and the implanted lead.

Any of the aspects herein, wherein generating the lead parameter comprises: determining a first pose of the initial lead relative to the target anatomical element in the first image and a second pose of the implanted lead relative to the target anatomical element in the second image; determining a difference between the first pose and the second pose; and applying the difference to the initial lead parameter to generate the implanted lead parameter.

Any of the aspects herein, wherein the memory stores further data for processing by the processor that, when processed, causes the processor to: receive a third image of the anatomical region, the third image depicting the anatomical element and at least a portion of the implanted lead; and generate an updated implanted lead parameter for the lead based on a combination of the implanted lead parameter and a correlation of the second image with the third image.

Any of the aspects herein, wherein generating the updated implanted lead parameter comprises: determining a first pose of the implanted lead relative to the target anatomical element in the second image and a second pose of the implanted lead relative to the target anatomical element in the third image; determining a difference between the first pose and the second pose; and applying the difference to the implanted lead parameter to generate the updated implanted lead parameter.

Any of the aspects herein, wherein the first image comprises one or more representations of an X-ray image of the anatomical region and the initial lead, and wherein the memory stores further data for processing by the processor that, when processed, causes the processor to: segment the one or more representations to identify the anatomical element and the initial lead.

Any of the aspects herein, wherein the first image comprises a plurality of representations of the X-ray image of the anatomical region and the initial lead, and wherein the memory stores further data for processing by the processor that, when processed, causes the processor to: combine the plurality of representations to form a single representation.

Any of the aspects herein, wherein the memory stores further data for processing by the processor that, when processed, causes the processor to: generate a trajectory for placing the implanted lead near the anatomical element.

Any of the aspects herein, wherein the anatomical region comprises at least one of a spinal region or a cranial region.

Any of the aspects herein, wherein the pulse generator is implantable.

A system for generating a lead parameter according to at least one embodiment of the present disclosure comprises a processor; and a memory storing data for processing by the processor, the data, when processed, causes the processor to: receive a first image of an anatomical region, wherein the first image depicts an anatomical element and at least a portion of an initial lead; process the first image to identify the anatomical element and the initial lead; receive an initial lead parameter associated with the initial lead; receive a second image of the anatomical region, wherein the second image depicts at least a portion of the anatomical element and at least a portion of an implanted lead, the second image taken at a different time than the first image; process the second image to identify the anatomical element and the implanted lead; and generate an implanted lead parameter for the lead based on a combination of the initial lead parameter, a correlation of the anatomical element of the first image with the anatomical element of the second image, and a correlation of the initial lead of the first image with the implanted lead of the second image.

Any of the aspects herein, wherein the first image comprises at least one of an optical image, a fluoroscopic image, an x-ray image, or a three-dimensional image.

Any of the aspects herein, wherein the second image comprises at least one of an optical image, an x-ray image, or a three-dimensional image.

Any of the aspects herein, wherein the memory stores further data for processing by the processor that, when processed, causes the processor to: generate a trajectory for placing the implanted lead near the anatomical element.

Any of the aspects herein, wherein the memory stores further data for processing by the processor that, when processed, causes the processor to: process the first image to identify the target anatomical element and the initial lead; and process the second image to identify the target anatomical element and the implanted lead.

Any of the aspects herein, wherein generating the implanted lead parameter comprises: determining a first pose of the initial lead relative to the target anatomical element in the first image and a second pose of the implanted lead relative to the target anatomical element in the second image; determining a difference between the first pose and the second pose; and applying the difference to the initial lead parameter to generate the implanted lead parameter.

Any of the aspects herein, wherein the memory stores further data for processing by the processor that, when processed, causes the processor to: receive a third image of the anatomical region, the third image depicting the anatomical element and at least a portion of the implanted lead; and generate an updated implanted lead parameter for the implanted lead based on a combination of the implanted lead parameter and a correlation of the second image with the third image.

Any of the aspects herein, wherein generating the updated implanted lead parameter comprises: determining a first pose of the implanted lead relative to the target anatomical element in the second image and a second pose of the implanted lead relative to the target anatomical element in the third image; determining a difference between the first pose and the second pose; and applying the difference to the implanted lead parameter to generate the updated lead parameter.

A system for generating a lead parameter according to at least one embodiment of the present disclosure comprises a processor; and a memory storing data for processing by the processor, the data, when processed, causes the processor to: receive an optical image of an anatomical region comprising an anatomical element and an initial lead positioned near the anatomical element; process the optical image to identify the anatomical element and the initial lead; receive an initial lead parameter associated with the initial lead; receive an additional image of the anatomical region and an implanted lead positioned near the anatomical element; process the additional image to identify the anatomical element and the implanted lead; correlate the identified anatomical element and the initial lead of the optical image and the identified anatomical element and the implanted lead of the additional image; and generate an implanted lead parameter based on the correlation and the initial lead parameter.

Any aspect in combination with any one or more other aspects.

Any one or more of the features disclosed herein.

Any one or more of the features as substantially disclosed herein.

Any one or more of the features as substantially disclosed herein in combination with any one or more other features as substantially disclosed herein.

Any one of the aspects/features/embodiments in combination with any one or more other aspects/features/embodiments.

Non-claimed use of any one or more of the aspects or features as disclosed herein.

It is to be appreciated that any feature described herein can be claimed in combination with any other feature(s) as described herein, regardless of whether the features come from the same described embodiment.

The details of one or more aspects of the disclosure are set forth in the accompanying drawings and the description below. Other features, objects, and advantages of the techniques described in this disclosure will be apparent from the description and drawings, and from the claims.

The phrases "at least one", "one or more", and "and/or" are open-ended expressions that are both conjunctive and disjunctive in operation. For example, each of the expressions "at least one of A, B and C", "at least one of A, B, or C", "one or more of A, B, and C", "one or more of A, B, or C" and "A, B, and/or C" means A alone, B alone, C alone, A and B together, A and C together, B and C together, or A, B and C together. When each one of A, B, and C in the above expressions refers to an element, such as X, Y, and Z, or class of elements, such as X1-Xn, Y1-Ym, and Z1-Zo, the phrase is intended to refer to a single element selected from X, Y, and Z, a combination of elements selected from the same class (e.g., X1 and X2) as well as a combination of elements selected from two or more classes (e.g., Y1 and Zo).

The term "a" or "an" entity refers to one or more of that entity. As such, the terms "a" (or "an"), "one or more" and "at least one" can be used interchangeably herein. It is also to be noted that the terms "comprising", "including", and "having" can be used interchangeably.

The preceding is a simplified summary of the disclosure to provide an understanding of some aspects of the disclosure. This summary is neither an extensive nor exhaustive overview of the disclosure and its various aspects, embodiments, and configurations. It is intended neither to identify key or critical elements of the disclosure nor to delineate the scope of the disclosure but to present selected concepts of the disclosure in a simplified form as an introduction to the more detailed description presented below. As will be appreciated, other aspects, embodiments, and configurations of the disclosure are possible utilizing, alone or in combination, one or more of the features set forth above or described in detail below.

Numerous additional features and advantages of the present disclosure will become apparent to those skilled in the art upon consideration of the embodiment descriptions provided hereinbelow.

### BRIEF DESCRIPTION OF THE SEVERAL VIEWS OF THE DRAWINGS

The accompanying drawings are incorporated into and form a part of the specification to illustrate several examples of the present disclosure. These drawings, together with the description, explain the principles of the disclosure. The drawings simply illustrate preferred and alternative examples of how the disclosure can be made and used and are not to be construed as limiting the disclosure to only the illustrated and described examples. Further features and advantages will become apparent from the following, more detailed, description of the various aspects, embodiments, and configurations of the disclosure, as illustrated by the drawings referenced below.
Fig. 1 is a block diagram of a therapeutic neuromodulation system according to at least one embodiment of the present disclosure;
Fig. 2 is a block diagram of a system according to at least one embodiment of the present disclosure;
Fig. 3 is a flowchart according to at least one embodiment of the present disclosure;
Fig. 4 is a flowchart according to at least one embodiment of the present disclosure; and
Fig. 5 is a flowchart according to at least one embodiment of the present disclosure.

### DETAILED DESCRIPTION

It should be understood that various aspects disclosed herein may be combined in different combinations than the combinations specifically presented in the description and accompanying drawings. It should also be understood that, depending on the example or embodiment, certain acts or events of any of the processes or methods described herein may be performed in a different sequence, and/or may be added, merged, or left out altogether (e.g., all described acts or events may not be necessary to carry out the disclosed techniques according to different embodiments of the present disclosure). In addition, while certain aspects of this disclosure are described as being performed by a single module or unit for purposes of clarity, it should be understood that the techniques of this disclosure may be performed by a combination of units or modules associated with, for example, a computing device and/or a medical device.

In one or more examples, the described methods, processes, and techniques may be implemented in hardware, software, firmware, or any combination thereof. If implemented in software, the functions may be stored as one or more instructions or code on a computer-readable medium and executed by a hardware-based processing unit. Alternatively or additionally, functions may be implemented using machine learning models, neural networks, artificial neural networks, or combinations thereof (alone or in combination with instructions). Computer-readable media may include non-transitory computer-readable media, which corresponds to a tangible medium such as data storage media (e.g., RAM, ROM, EEPROM, flash memory, or any other medium that can be used to store desired program code in the form of instructions or data structures and that can be accessed by a computer).

Instructions may be executed by one or more processors, such as one or more digital signal processors (DSPs), general purpose microprocessors (e.g., Intel Core i3, i5, i7, or i9 processors; Intel Celeron processors; Intel Xeon processors; Intel Pentium processors; AMD Ryzen processors; AMD Athlon processors; AMD Phenom processors; Apple A10 or 10X Fusion processors; Apple A11, A12, A12X, A12Z, or A13 Bionic processors; or any other general purpose microprocessors), graphics processing units (e.g., Nvidia GeForce RTX 2000-series processors, Nvidia GeForce RTX 3000-series processors, AMD Radeon RX 5000-series processors, AMD Radeon RX 6000-series processors, or any other graphics processing units), application specific integrated circuits (ASICs), field programmable logic arrays (FPGAs), or other equivalent integrated or discrete logic circuitry. Accordingly, the term "processor" as used herein may refer to any of the foregoing structure or any other physical structure suitable for implementation of the described techniques. Also, the techniques could be fully implemented in one or more circuits or logic elements.

Before any embodiments of the disclosure are explained in detail, it is to be understood that the disclosure is not limited in its application to the details of construction and the arrangement of components set forth in the following description or illustrated in the drawings. The disclosure is capable of other embodiments and of being practiced or of being carried out in various ways. Also, it is to be understood that the phraseology and terminology used herein is for the purpose of description and should not be regarded as limiting. The use of "including," "comprising," or "having" and variations thereof herein is meant to encompass the items listed thereafter and equivalents thereof as well as additional items. Further, the present disclosure may use examples to illustrate one or more aspects thereof. Unless explicitly stated otherwise, the use or listing of one or more examples (which may be denoted by "for example," "by way of example," "e.g.," "such as," or similar language) is not intended to and does not limit the scope of the present disclosure.

The terms proximal and distal are used in this disclosure with their conventional medical meanings, proximal being closer to the operator or user of the system, and further from the region of surgical interest in or on the patient, and distal being closer to the region of surgical interest in or on the patient, and further from the operator or user of the system.

As previously described, in closed-loop neuromodulation therapies, an electrical signal generated by a pulse generator may be sent to a stimulation target. A resulting signal from the electrical signal may be recorded. The resulting signal may provide information by which to adjust the electrical signal. For example, spinal cord stimulation (SCS) is a form of closed-loop neuromodulation in which contacts are placed in the epidural space near the patient's spinal cord and one or more nerves are stimulated to block pain signal(s) sent to the brain from the one or more nerves. SCS can be used to alleviate, for example, back pain, and can help mask or alleviate back pain.

Modern SCS stimulation patterns are based on specific anatomical locations. In order to guide initial programming, a segmented X-ray (or an X-ray having key points identified) can be used to provide the initial setup of the stimulation pattern. Additionally, a test implantation of the lead(s) is often performed before the permanent implantation to ensure that the neuromodulation therapy will be effective. The test implantation may use wires or other components that are external to the user so that one or more parameters of the test leads can be adjusted during the test. Once the test leads are inserted, programmed, and deemed effective, the test leads are removed and the implanted leads are inserted and programmed. The implanted leads may or may not have external wires or components. However, the process to program the SCS leads (or any lead of any neuromodulation therapy) can be a time-consuming, trial and error process. If the test leads have been effectively programmed, it would be desirable to transfer this program to the new implanted leads. However, the implanted leads will not be positioned in the same position, therefore, it is difficult to transfer the test lead programs and parameters as the leads will have changed positions. In addition, sometimes leads can move positions after being placed. If this happens, it would be desirable to transfer or translate the existing lead program to the new positions of the leads.

In at least one embodiment of the present disclosure, images of a spinal region of a patient can be used to inform an automated or semi-automated lead programming algorithm during placement of the implanted lead. In some embodiments where the images are X-ray images or fluoroscopic images, it will be appreciated that anatomical elements such as vertebral bone are visible in the images and a position of a spinal cord of the patient (which may not be visible) can be inferred from the visible vertebral bone. An image registration or correlation can be used to align previous images to new images and the registration can facilitate aligning, for example, vertebrae segmentations, anatomy, and calculating new lead stimulation parameters in an attempt to recreate the previous or initial stimulation parameters. Further, geometric algorithms can be used along with current steering capabilities in modern spinal cord stimulators to match a desired location of anode and cathodes with respect to the spinal cord.

The images described above in connection with lead placement may include X-ray images, fluoroscopic images, three-dimensional (3D) images, and/or 2D images. However, obtaining one or more of the images (e.g., X-ray images) may be time consuming and difficult due to setting up picture archiving and communication system (PACS) servers and clients, establishing security protocols, and business agreements around protected health information (PHI) for transferring 2D medical images from a hospital or clinic to a physician programmer. Such processes are difficult and can slow down implementation of the automatic lead programming system described above.

In at least one embodiment of the present disclosure, an optical camera can be used to capture a representation of the image on a computer display. Image analysis can be done on this captured image and the analysis features may be saved. The above-described capture and analysis process may circumvent setting up PACS servers and clients and establishing network security protocols. Further, the process may not save the raw image, which removes the need to set up agreements specifically for PHI (or any other agreement that may be necessary) and reduces the amount of memory needed to store the analysis features. In order to transfer the best image possible, multiple images may be captured and combined using a variety of image processing techniques (e.g., averaging, summing, etc.). Additionally, multiple images can be captured and their segmentations (or other image processed data) may be combined using a variety of image or signal processing techniques (e.g., averaging, summing, etc.). Further, to help correct for skew, optical image processing and analysis techniques can be used or a depth camera aligned and calibrated with the optical camera could be used to correct in image processing or prompt the user to correct in the physical space the alignment of the camera to the displayed image.

A camera (such as, for example, a smartphone camera) used by the physician programmer to visualize the X-ray of the implanted lead and segment it could reduce programming time for the physician and/or a sales representative. Additionally, machine learning or AI can be used to segment the image for automatic SCS programming.

Thus, embodiments of the present disclosure provide technical solutions to one or more of the problems of (1) transferring and/or adjusting lead parameters of a test lead during testing to a lead permanently implanted in a patient, (2) adjusting lead parameters of the lead when the lead has moved, (3) obtaining information of a lead placement from representations of an image, and (4) increasing patient comfort and safety.

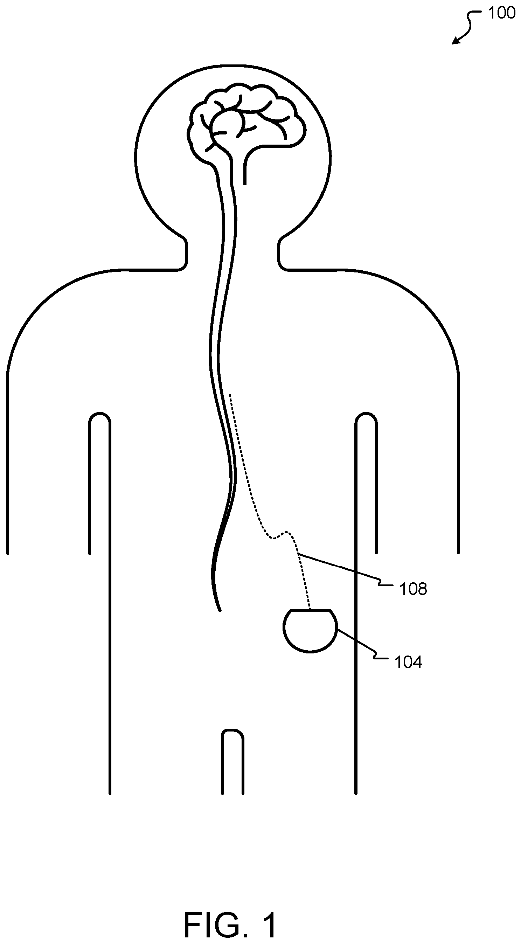

Turning to Fig. 1, a diagram of aspects of a therapeutic neuromodulation system 100 according to at least one embodiment of the present disclosure is shown. The therapeutic neuromodulation system 100 may be used to provide electric signals for a patient and/or carry out one or more other aspects of one or more of the methods disclosed herein. In some examples, the device 104 may be referred to as a pulse generator. The pulse generator may be implantable in a patient or may be external to the patient. Additionally, the therapeutic neuromodulation system 100 may include one or more wires or leads 108 that provide a connection between the device 104 and nerves of the patient for enabling the stimulation/blocking therapy.

Neuromodulation techniques (e.g., technologies that act directly upon nerves of a patient, such as the alteration, or "modulation," of nerve activity by delivering electrical impulses or pharmaceutical agents directly to a target area) may be used for assisting in treatments for different diseases, disorders, or ailments of a patient, such as chronic pain. Accordingly, as described herein, the neuromodulation techniques may be used for blocking pain signals sent to a patient's brain to relieve chronic pain. For example, the device 104 may provide electrical stimulation to one or more nerves in the spinal cord of the patient (e.g., via the one or more leads 108) to block the pain signals from being received by the brain. In other examples, the device 104 may provide electrical stimulation to the brain.

In some examples, as shown in Fig. 1, the one or more leads 108 may include one lead 108. In other embodiments, the one or more leads 108 may include multiple leads. The lead 108 may be implanted on or near any anatomical element targeted for therapy. In some examples, the lead 108 is implanted near the spinal cord and more specifically, in the epidural space between the spinal cord and the vertebrae. Once implanted, the lead 108 may provide an electrical signal (whether stimulating or blocking) from the device 104 to the anatomical element (e.g., one or more nerves in the spinal cord, the brain, etc.). The device 104 in some embodiments, may be implanted in the patient, though in other embodiments - such as during testing of the lead 108 - the device 104 may be external to the patient's body.

In some examples, the lead 108 may provide the electrical signals to the respective nerves via electrodes that are connected to the nerves (e.g., sutured in place, wrapped around the nerves, etc.). In some examples, the lead 108 may be referenced as cuff electrodes or may otherwise include the cuff electrodes (e.g., at an end of the lead 108 not connected or plugged into the device 104). Additionally or alternatively, while shown as physical wires that provide the connection between the device 104 and the one or more nerves, the electrodes may provide the electrical signals to the one or more nerves wirelessly (e.g., with or without the device 104).

Additionally, while not shown, the therapeutic neuromodulation system 100 may include one or more processors (e.g., one or more DSPs, general purpose microprocessors, graphics processing units, ASICs, FPGAs, or other equivalent integrated or discrete logic circuitry) that are programmed to carry out one or more aspects of the present disclosure. In some examples, the one or more processors may be used to control one or more parameters of the lead 108. In other examples, the one or more processors may include a memory or may be otherwise configured to perform the aspects of the present disclosure. For example, the one or more processors may provide instructions to the device 104, the electrodes, or other components of the therapeutic neuromodulation system 100 not explicitly shown or described with reference to Fig. 1 for providing the pain blocking therapy to regulate chronic pain in a patient as described herein. In some examples, the one or more processors may be part of the device 104 or part of a control unit for the therapeutic neuromodulation system 100 (e.g., where the control unit is in communication with the device 104 and/or other components of the therapeutic neuromodulation system 100).

The therapeutic neuromodulation system 100 or similar systems may be used, for example, to carry out one or more aspects of any of the methods 300, 400, and/or 500 described below. In some embodiments, the therapeutic neuromodulation system 100 may be used with a system 200 for programming the lead 108 based on a location of and initial parameters of an initial lead. The therapeutic neuromodulation system 100 or similar systems may also be used for other purposes. It will be appreciated that the human body has many nerves and the stimulation or blocking therapies described herein may be applied to any one or more nerves, which may reside at any location of a patient (e.g., lumbar, thoracic, extremity, brain, trunk etc.). The stimulation or blocking therapies may also be applied to any stimulation target (e.g., an anatomical element, non-neuronal cells, etc.)

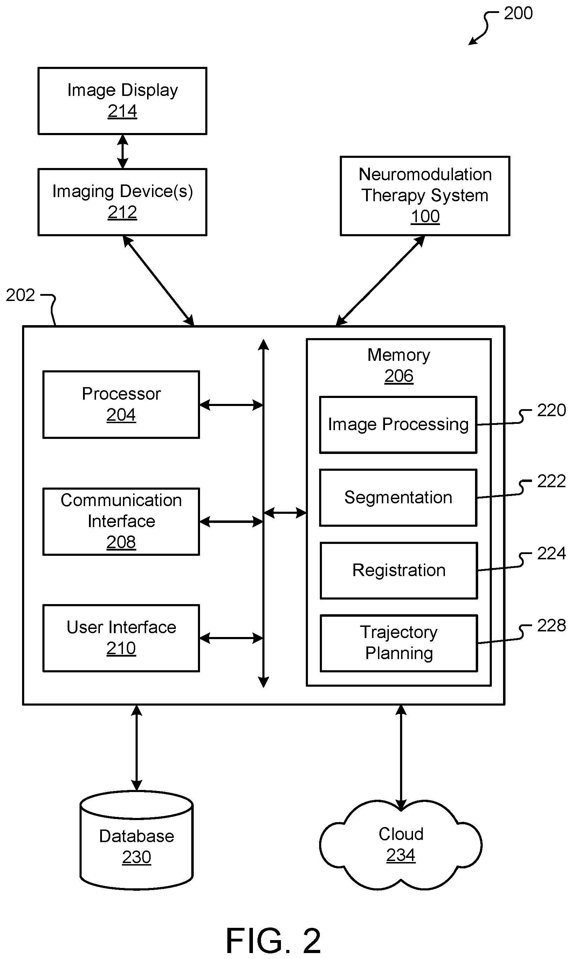

Turning to Fig. 2, a block diagram of a system 200 according to at least one embodiment of the present disclosure is shown. The system 200 may be used to for controlling the therapeutic neuromodulation system 100, for programing one or more leads 108 of the therapeutic neuromodulation system 100, and/or to carry out one or more other aspects of one or more of the methods disclosed herein. The system 200 comprises a computing device 202, the therapeutic neuromodulation system 100, one or more imaging devices 212, a database 230, and/or a cloud or other network 234. Systems according to other embodiments of the present disclosure may comprise more or fewer components than the system 200. For example, the system 200 may not include the imaging device 212, one or more components of the computing device 202, the database 230, and/or the cloud 234.

The computing device 202 comprises a processor 204, a memory 206, a communication interface 208, and a user interface 210. Computing devices according to other embodiments of the present disclosure may comprise more or fewer components than the computing device 202.

The processor 204 of the computing device 202 may be any processor described herein or any similar processor. The processor 204 may be configured to execute instructions stored in the memory 206, which instructions may cause the processor 204 to carry out one or more computing steps utilizing or based on data received from the imaging device 212, the therapeutic neuromodulation system 100, the database 230, and/or the cloud 234.

The memory 206 may be or comprise RAM, DRAM, SDRAM, other solid-state memory, any memory described herein, or any other tangible, non-transitory memory for storing computer-readable data and/or instructions. The memory 206 may store information or data useful for completing, for example, any step of the methods 300, 400, and/or 500 described herein, or of any other methods. The memory 206 may store, for example, instructions and/or machine learning models that support one or more functions of the therapeutic neuromodulation system 100. For instance, the memory 206 may store content (e.g., instructions and/or machine learning models) that, when executed by the processor 204, enable image processing 220, segmentation 222, registration 224, and/or trajectory planning 228. Such content, if provided as in instruction, may, in some embodiments, be organized into one or more applications, modules, packages, layers, or engines.

The image processing 220 enables the processor 204 to process image data of an image (obtained from, for example, the imaging device 212, the user interface 210, the communication interface 208, the database 230, and/or the cloud 234) for the purpose of, for example, identifying information about anatomical elements and/or objects such as the lead(s) 108 depicted in the image. The information may comprise, for example, identification of hard tissue and/or soft tissues, a boundary between hard tissue and soft tissue, a boundary of hard tissue and/or soft tissue, identification of a lead such as the lead 108, etc. The image processing 220 may, for example, identify hard tissue, soft tissue, the lead 108, and/or a boundary of the hard tissue and/or soft tissue by determining a difference in or contrast between colors or grayscales of image pixels. For example, a boundary between the hard tissue and the soft tissue may be identified as a contrast between lighter pixels and darker pixels. The imaging processing 220 may also use segmentation 222, as described below.

The image processing 220 may also enable the processor 204 to combine one or more images, representations of images (e.g., an image of an image), and/or sets of image data. The image processing 220 may combine the one or more images, representations of images, and/or sets of image data by averaging the one or more images, representations of images, and/or sets of image data to form a single image, representation of an image, or set of image data.

The segmentation 222 enables the processor 204 to segment the image data so as to identify individual objects and/or anatomical elements in the image. The segmentation 222 may enable the processor 204 to identify a boundary of an object or an anatomical element by using, for example, feature recognition. For example, the segmentation 222 may enable the processor 204 to identify a vertebra in image data. In other instances, the segmentation 222 may enable the processor 204 to identify a boundary of an object such as, for example, the lead 108 or an anatomical element by determining a difference in or contrast between colors or grayscales of image pixels.

The registration 224 enables the processor 204 to correlate an image with another image. The registration 224 may enable the processor 204 to also correlate identified anatomical elements and/or individual objects in one image with identified anatomical elements and/or individual objects in another image. The registration 224 may enable information about the anatomical elements and the individual objects (such as, for example, the lead 108) to be obtained and measured. For example, a distance of an initial lead (e.g., a test lead) to the anatomical element in a first image and a distance of a lead (e.g., an implanted lead) to the anatomical element in a second image can be determined by the processor 204. This information (among other inputs) can be used in determining lead parameters for the lead.

The trajectory planning 228 enables the processor 204 to determine a trajectory for a lead such as the lead 108 (whether a test lead, an implanted lead, and/or a permanent lead) to position the lead 108 at a desired pose (e.g., position and orientation) within a patient. The processor 204 may receive the desired pose, information about the anatomical region, information about the instruments and/or tools for positioning the lead, etc. as input for the trajectory planning 228, which may then determine an optimal trajectory for the lead. The trajectory may avoid critical features (e.g., nerves or the like) and/or prioritize shorter trajectories.

The memory 206 may store other types of content or data (e.g., machine learning models, artificial neural networks, deep neural networks, etc.) that can be processed by the processor 204 to carry out the various method and features described herein. Thus, although various contents of memory 206 may be described as instructions, it should be appreciated that functionality described herein can be achieved through use of instructions, algorithms, and/or machine learning models. The data, algorithms, and/or instructions may cause the processor 204 to manipulate data stored in the memory 206 and/or received from or via the imaging device 212, the therapeutic neuromodulation system 100, the database 230, and/or the cloud 234.

The computing device 202 may also comprise a communication interface 208. The communication interface 208 may be used for receiving image data or other information from an external source (such as the imaging device 212, the therapeutic neuromodulation system 100, the database 230, the cloud 234, and/or any other system or component not part of the system 200), and/or for transmitting instructions, images, or other information to an external system or device (e.g., another computing device 202, the imaging device 212, the therapeutic neuromodulation system 100, the database 230, the cloud 234, and/or any other system or component not part of the system 200). The communication interface 208 may comprise one or more wired interfaces (e.g., a USB port, an Ethernet port, a Firewire port) and/or one or more wireless transceivers or interfaces (configured, for example, to transmit and/or receive information via one or more wireless communication protocols such as 802.11a/b/g/n, Bluetooth, NFC, ZigBee, and so forth). In some embodiments, the communication interface 208 may be useful for enabling the device 202 to communicate with one or more other processors 204 or computing devices 202, whether to reduce the time needed to accomplish a computing-intensive task or for any other reason.

The computing device 202 may also comprise one or more user interfaces 210. The user interface 210 may be or comprise a keyboard, mouse, trackball, monitor, television, screen, touchscreen, and/or any other device for receiving information from a user and/or for providing information to a user. The user interface 210 may be used, for example, to receive a user selection or other user input regarding any step of any method described herein. Notwithstanding the foregoing, any required input for any step of any method described herein may be generated automatically by the system 200 (e.g., by the processor 204 or another component of the system 200) or received by the system 200 from a source external to the system 200. In some embodiments, the user interface 210 may be useful to allow a surgeon or other user to modify instructions to be executed by the processor 204 according to one or more embodiments of the present disclosure, and/or to modify or adjust a setting of other information displayed on the user interface 210 or corresponding thereto.

Although the user interface 210 is shown as part of the computing device 202, in some embodiments, the computing device 202 may utilize a user interface 210 that is housed separately from one or more remaining components of the computing device 202. In some embodiments, the user interface 210 may be located proximate one or more other components of the computing device 202, while in other embodiments, the user interface 210 may be located remotely from one or more other components of the computer device 202.

The imaging device 212 may be operable to image anatomical feature(s) (e.g., a bone, veins, tissue, etc.), objects such as the lead 108, and/or other aspects of patient anatomy or anatomical region to yield image data (e.g., image data depicting or corresponding to a bone, veins, tissue, etc.). "Image data" as used herein refers to the data generated or captured by an imaging device 212, including in a machine-readable form, a graphical/visual form, and in any other form. In various examples, the image data may comprise data corresponding to an anatomical feature of a patient, or to a portion thereof. In some embodiments, a first imaging device 212 may be used to obtain first image data (e.g., a first image) at a first time, and a second imaging device 212 may be used to obtain second image data (e.g., a second image) at a second time after the first time. The imaging device 212 may be capable of taking a 2D image or a 3D image to yield the image data. The imaging device 212 may be or comprise, for example, an ultrasound scanner (which may comprise, for example, a physically separate transducer and receiver, or a single ultrasound transceiver), an O-arm, a C-arm, a G-arm, or any other device utilizing X-ray-based imaging (e.g., a fluoroscope, a CT scanner, or other X-ray machine), a magnetic resonance imaging (MRI) scanner, an optical coherence tomography (OCT) scanner, an endoscope, a microscope, an optical camera, a thermographic camera (e.g., an infrared camera), a radar system (which may comprise, for example, a transmitter, a receiver, a processor, and one or more antennae), or any other imaging device 212 suitable for obtaining images of an anatomical feature and/or leads implanted or positioned in a patient. The imaging device 212 may be contained entirely within a single housing, or may comprise a transmitter/emitter and a receiver/detector that are in separate housings or are otherwise physically separated.

In some embodiments, the imaging device 212 may comprise one or more than one imaging device 212. For example, a first imaging device may provide first image data and/or a first image, and a second imaging device may provide second image data and/or a second image. In still other embodiments, the same imaging device may be used to provide both the first image data and the second image data, and/or any other image data described herein. The imaging device 212 may be operable to generate a stream of image data. For example, the imaging device 212 may be configured to operate with an open shutter, or with a shutter that continuously alternates between open and shut so as to capture successive images. For purposes of the present disclosure, unless specified otherwise, image data may be considered to be continuous and/or provided as an image data stream if the image data represents two or more frames per second.

The database 230 may store information such as patient data, results of a stimulation and/or blocking procedure, stimulation and/or blocking parameters, electrical signal parameters, electrode parameters, etc. The database 230 may be configured to provide any such information to the computing device 202 or to any other device of the system 200 or external to the system 200, whether directly or via the cloud 234. In some embodiments, the database 230 may be or comprise part of a hospital image storage system, such as the PACS, the HIS, and/or another system for collecting, storing, managing, and/or transmitting electronic medical records.

The cloud 234 may be or represent the Internet or any other wide area network. The computing device 202 may be connected to the cloud 234 via the communication interface 208, using a wired connection, a wireless connection, or both. In some embodiments, the computing device 202 may communicate with the database 230 and/or an external device (e.g., a computing device) via the cloud 234.

The system 200 or similar systems may be used, for example, to carry out one or more aspects of any of the non-claimed methods 300, 400, and/or 500 described herein. The system 200 or similar systems may also be used for other purposes.

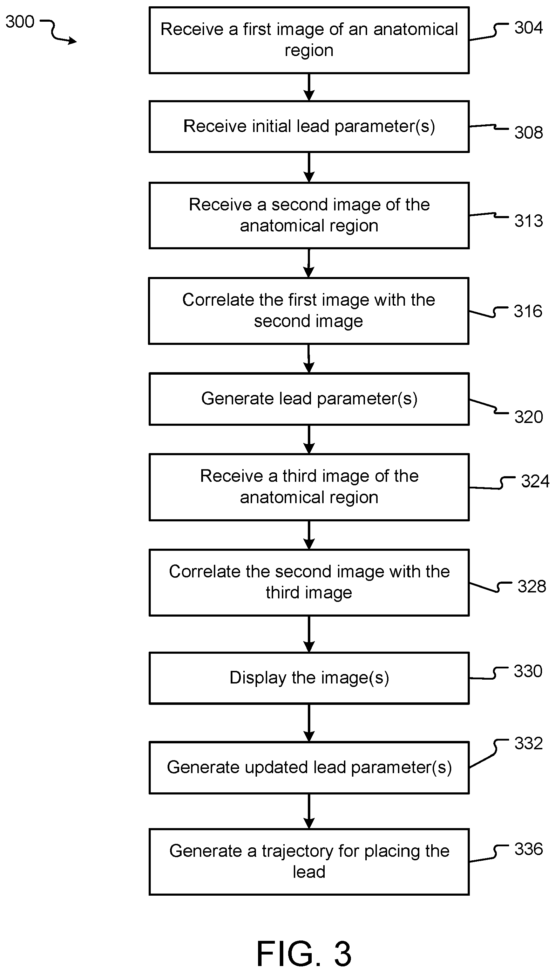

Fig. 3 depicts a non-claimed method 300 that may be used, for example, for generating lead parameter(s).

The method 300 (and/or one or more steps thereof) may be carried out or otherwise performed, for example, by at least one processor. The at least one processor may be the same as or similar to the processor(s) 204 of the computing device 202 described above. The at least one processor may be part of a therapeutic neuromodulation system such as the therapeutic neuromodulation system 100. A processor other than any processor described herein may also be used to execute the method 300. The at least one processor may perform the method 300 by executing elements stored in a memory such as the memory 206. The elements stored in the memory and executed by the processor may cause the processor to execute one or more steps of a function as shown in method 300. One or more portions of a method 300 may be performed by the processor executing any of the contents of memory, such as an image processing 220, a segmentation 222, a registration 224, and/or a trajectory planning 228.

The method 300 comprises receiving a first image of an anatomical region (step 304). The first image may be of an anatomical region and may depict at least one anatomical element and at least a portion of an initial lead (e.g., a test lead) that may be the same as or similar to the lead 108. The anatomical region may be, for example, a spinal region, a cranial region, or any region of a patient. The anatomical region may also comprise a target anatomical element that is easily identifiable in relation to the lead. For example, the target anatomical element may comprise a vertebra that is easily identifiable in images and the anatomical element may comprise a spinal cord that receives electrical stimulation transmitted by the lead. More specifically, where the images are X-ray images or fluoroscopic images, it will be appreciated that target anatomical elements such as vertebral bone are visible in the images and a position of an anatomical element that may not be visible such as a spinal cord of the patient can be inferred from the visible vertebral bone.

The first image may be received via a user interface such as the user interface 210 and/or a communication interface such as the communication interface 208 of a computing device such as the computing device 202, and may be stored in a memory such as the memory 206 of the computing device. The first image may also be received from an external database or image repository (e.g., a hospital image storage system, such as the PACS, the HIS, and/or another system for collecting, storing, managing, and/or transmitting electronic medical records including image data), and/or via the Internet or another network. In other embodiments, the first image may be received or obtained from an imaging device such as the imaging device 212, which may be any imaging device such as an optical camera, an MRI scanner, a CT scanner, any other X-ray based imaging device, or an ultrasound imaging device. The image may also be generated by and/or uploaded to any other component of a system such as the system 200. In some embodiments, the image may be indirectly received via any other component of the system or a node of a network to which the system is connected.

The first image may be a 2D image or a 3D image or a set of 2D and/or 3D images. The image may depict the anatomical region. In some embodiments, the first image may comprise a representation of an X-ray image displayed on a display (such as, for example, a user interface such as the user interface 210). The representation may be obtained from the imaging device and in some embodiments the imaging device may be an optical camera (e.g., a smart phone camera, a point and shoot camera, a DSLR camera, etc.). In other words, the first image may be an optical image of an X-ray image. In other embodiments, the first image may comprise an X-ray image. In some embodiments, the first image may be captured prior to implantation of an implanted lead, which may be the same as or similar to the lead 108, and may be stored in a system (e.g., a system 200) and/or one or more components thereof (e.g., a database 230). The stored image may then be received (e.g., by a processor 204). In some embodiments, the first image may include incidental anatomical elements (e.g., ribs or other anatomical objects) in addition to target anatomical elements (e.g., vertebrae or other anatomical objects). The first image may comprise various features corresponding to the patient's anatomy and/or anatomical elements (and/or portions thereof), including gradients corresponding to boundaries and/or contours of the various depicted anatomical elements, varying levels of intensity corresponding to varying surface textures of the various depicted anatomical elements, combinations thereof, and/or the like. The first image may depict any portion or part of patient anatomy and may include, but is in no way limited to, one or more vertebrae, ribs, lungs, soft tissues (e.g., skin, tendons, muscle fiber, etc.), a patella, a clavicle, a scapula, combinations thereof, and/or the like.

The method 300 also comprises receiving initial lead parameter(s) (step 308). The initial lead parameter may be received via the user interface and/or the communication interface of the computing device, and may be stored in the memory of the computing device. The initial lead parameters may also be received from an external source such as the Internet or other network. The initial lead parameters may be obtained during a testing of the initial leads. The initial lead parameters may comprise one or more of the following: stimulation and/or blocking parameters, electrical signal parameters, electrode parameters, pulse width, pulse amplitude, stimulation direction, frequency, cycling time, biphasic stimulation, triphasic stimulation, monophasic stimulation, etc.

The method 300 also comprises receiving a second image of an anatomical region (step 312). The step 312 may be the same as or similar to the step 304 described above. The second image may be of the anatomical region and may depict the at least one anatomical element and at least a portion of a lead, which may also be referred to as an implanted lead, which may be the same as or similar to the lead 108. The second image may be taken at a different time than the first image. For example, the second image may be taken after the first image such as during implantation of the lead after a testing of the lead (wherein the first image is obtained at the testing of the lead).

The method 300 also comprises correlating the first image with the second image (step 316). The first image and the second image may be correlated with each other by a processor such as the processor 204 executing a registration such as the registration 224. The registration enables the processor to correlate an image (e.g., the first image) with another image (e.g., the second image). The registration may enable the processor to also correlate identified anatomical elements and/or individual objects (e.g., the lead) in one image with identified anatomical elements and/or individual objects in another image. The registration may enable information about the anatomical elements and the individual objects to be obtained and measured. For example, a distance of the initial lead (e.g., a test lead) to a target anatomical element in the first image and a distance of a lead (e.g., an implanted lead) to the target anatomical element in the second image can be determined by the processor.

The method 300 also comprises generating lead parameter(s) for the lead (step 320). The lead parameter may be referred to as an implanted lead parameter. The lead parameter may be generated by the processor of the computing device based on a combination of the initial lead parameter (received in, for example, the step 308) and the correlation of the first image and the second image (obtained from, for example, the step 312). It will be appreciated that the lead may be installed at a position and/or orientation that is different from a position and/or orientation of the initial lead during testing. Thus, new lead parameters are generated to compensate for a change in the position and/or orientation of the lead relative to the position and/or orientation of the initial lead. Thus, the generated lead parameter replicates results of the initial lead parameters in the instances where the lead is positioned at a different pose than the initial lead.

The method 300 also comprises receiving a third image of the anatomical region (step 324). The step 324 may be the same as or similar to the step 304 described above. The third image may be of the anatomical region and may depict the at least one anatomical element and at least a portion of the lead (e.g., the implanted lead). The third image may be taken at a different time than the first image and the second image. For example, the third image may be taken at a follow-up appointment after implantation of the lead (wherein the second image may have been taken during the implantation of the lead). The third image may be compared with the second image to determine, for example, if the lead has moved since implantation of the lead. In other examples, the third image - or any subsequent image - may be used to determine if the lead has moved as compared to a prior image.

The method 300 also comprises correlating the second image with the third image (step 328). The step 328 may be the same as or similar to the step 316. The third image may be correlated with the second image, though alternatively, the third image may be correlated with the first image.

The method 300 also comprises displaying the image(s) (step 330). Displaying the image(s) may include overlaying the first image, the second image, and/or the third image (or any combination thereof) and/or the segmentations so as to enable a user to visualize a difference between the segmentation or key point detection results. Displaying the images may also enable the user to view differences in the lead placement after implantation. For example, the user may be able to see that the lead has migrated after surgical implantation. The image(s) and/or overlay may be displayed on, for example, a user interface such as the user interface 210.

The method 300 also comprises generating updated lead parameter(s) (step 332). The step 332 may be the same as or similar to the step 320. The updated lead parameter may be generated based on the lead parameter (which may be generated in, for example, the step 320) and the correlation of the second image with the third image (which may be obtained from, for example, the step 328).

The steps 324, 328, and 332 may occur after the lead has been placed, for example, during a follow-up appointment with the patient. The steps 324, 328, and 332 may indicate that the lead has moved since the initial placement of the lead, thus, the lead parameter may need to be updated. The steps 324, 328, and 332 may also be repeated, as needed, during a lifetime of the lead. In other words, multiple images (e.g., more than three) can be received to repeat the steps 324, 328, and/or 332 as needed. In other instances, the method 300 may not include the steps 324, 328, and 332. In other words, the method 300 may receive two images to generate the lead parameter(s) and may end after the lead parameter(s) are generated.

The method 300 also comprises generating a trajectory for placing the lead (step 336). The trajectory may be generated by the processor executing a trajectory planning such as the trajectory planning 228. The trajectory planning enables the processor to determine a trajectory for lead to position the lead at a desired pose (e.g., position and orientation) within a patient. The processor may receive the desired pose, information about the anatomical region, information about the instruments and/or tools for positioning the lead, etc. as input for the trajectory planning, which may then determine an optimal trajectory for the lead. The trajectory may avoid critical features (e.g., nerves or the like) and/or prioritize shorter trajectories. The trajectory may comprise machine readable instructions for a robot or may comprise instructions for a user such as a medical provider or clinician.

It will be appreciated that the step 336 may occur after the step 320. In other words, the trajectory for placing the lead may occur after the lead parameter is generated. In other instances, the method 300 may not include the step 336.

The present disclosure encompasses embodiments of the method 300 that comprise more or fewer steps than those described above, and/or one or more steps that are different than the steps described above.

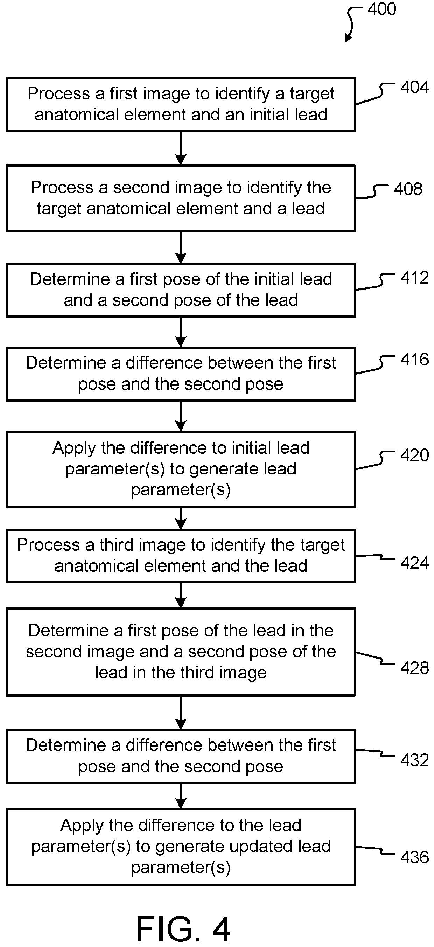

Fig. 4 depicts a non-claimed method 400 that may be used, for example, for determining lead parameter(s).

The method 400 (and/or one or more steps thereof) may be carried out or otherwise performed, for example, by at least one processor. The at least one processor may be the same as or similar to the processor(s) 204 of the computing device 202 described above. The at least one processor may be part of a therapeutic neuromodulation system such as the therapeutic neuromodulation system 100. A processor other than any processor described herein may also be used to execute the method 400. The at least one processor may perform the method 400 by executing elements stored in a memory such as the memory 206. The elements stored in the memory and executed by the processor may cause the processor to execute one or more steps of a function as shown in method 400. One or more portions of a method 400 may be performed by the processor executing any of the contents of memory, such as an image processing 220, a segmentation 222, a registration 224, and/or a trajectory planning 228.

The method 400 comprises processing a first image (step 404). The step 404 may be the same as or similar to the step 304 of the method 300 described above with respect to receiving an image. The first image may be of an anatomical region and may depict at least one anatomical element and at least a portion of an initial lead (e.g., a test lead) that may be the same as or similar to the lead 108. The anatomical region may also comprise a target anatomical element that is easily identifiable in relation to the lead. For example, the target anatomical element may comprise a vertebra, the anatomical element may comprise a spinal cord, and the lead may transmit the electrical signal to the spinal cord.

The first image may be processed using an image processing algorithm such as the image processing algorithm 220 to identify anatomical elements and/or the lead in the first image and in particular, to identify the target anatomical element and the initial lead. In some embodiments, feature recognition may be used to identify a feature of the target anatomical element or the initial lead. For example, a contour of a vertebrae, femur, or other bone may be identified in the first image. In other embodiments, the image processing algorithm may use artificial intelligence or machine learning to identify the target anatomical element and/or the initial lead. In such embodiments, a plurality of training images may be provided to a processor such as the processor 204, each training image annotated to include identifying information about a lead and/or an anatomical element in the image. The processor, executed instructions stored in memory such as the memory 206 or in another memory, may analyze the images using a machine-learning algorithm and, based on the analysis, generate one or more image processing algorithms such as the image processing algorithms for identifying anatomical elements and/or objects in an image.

The method 400 also comprises processing a second image (step 408). The step 408 may be the same as or similar to the step 404 described above. The second image may be of the anatomical region and may depict the at least one anatomical element, the target anatomical element, and at least a portion of a lead (e.g., an implanted lead), which may be the same as or similar to the lead 108. The second image may be processed to identify the target anatomical element and the lead.

The method 400 also comprises determining a first pose of an initial lead and a second pose of a lead (step 412). The first pose of the initial lead and the second pose of the lead may be identified relative to the target anatomical element. The first pose of the initial lead and the second pose of the lead may be identified by the processor using the image processing. In other examples, the processor may use a segmentation such as the segmentation 222 to identify and determine the first pose of the initial lead and the second pose of the lead. The segmentation enables the processor to segment the image data so as to identify individual objects and/or anatomical elements in the image. The segmentation may enable the processor to identify a boundary of an object or an anatomical element by using, for example, feature recognition. For example, the segmentation may enable the processor to identify a vertebra in image data. In other instances, the segmentation may enable the processor to identify a boundary of an object such as, for example, a lead such as the lead or an anatomical element by determining a difference in or contrast between colors or grayscales of image pixels. It will be appreciated that the processor may use the segmentation when processing the image in the steps 404, 408, and/or 424. It will also be appreciated that the processor may identify the initial lead, the lead, anatomical element(s), or any other key features using any image processing or process to identify such features.

The method 400 also comprises determining a difference between the first pose and the second pose (step 416). The difference may be calculated by subtracting one or more coordinates of the first pose from one or more coordinates of the second pose, or vice versa. The difference may also be obtained by overlaying the first image (whether a 2D image or a 3D image) over the second image and measuring a difference between the first pose and the second pose.

The method 400 also comprises applying the difference to initial lead parameter(s) to generate lead parameter(s) (step 420). The initial lead parameter may correspond to the initial lead (e.g., the test lead) and the lead parameter may correspond to the lead (e.g., the implanted lead). In some instances, the difference may be simply added to one or more parameters of the initial lead parameters to generate the lead parameter. In other instances, the difference may be used as input to determine a relative factor to apply to the initial lead parameter to generate the lead parameter.

The method 400 also comprises processing a third image (step 424). The step 424 may be the same as or similar to the step 404 described about. The third image may be of the anatomical region and may depict the at least one anatomical element, the target anatomical element, and/or at least a portion of the lead (e.g., the implanted lead). The third image may be obtained at a time different from the second image. For example, the third image may be taken at a follow-up appointment after implantation of the lead (wherein the second image may have been taken during the implantation of the lead). The third image may be compared with the second image to determine, for example, if the lead has moved since implantation of the lead. In other examples, the third image - or any subsequent image - may be used to determine if the lead has moved as compared to a prior image.

The method 400 also comprises determining a first pose of the lead in the second image and a second pose of the lead in the third image (step 428). The step 428 may be the same as or similar to the step 412 above. The first pose of the lead and the second pose of the lead may be identified relative to the target anatomical element.

The method 400 also comprises determining a difference between the first pose and the second pose (step 432). The step 432 may be the same as or similar to the step 420 above.

The method 400 also comprises applying the difference to the lead parameter(s) to generate an updated lead parameter(s) (step 436). The step 436 may be the same as or similar to the step 420 above.

The steps 424, 428, 432, and/or 436 may occur after the lead has been placed, for example, during a follow-up appointment with the patient. The steps 424, 428, 432, and/or 436 may indicate that the lead has moved since the initial placement of the lead, thus, the lead parameter(s) may need to be updated. The steps 424, 428, 432, and/or 436 may also be repeated, as needed, during a lifetime of the lead. In other words, multiple images (e.g., more than three) can be processed to repeat the steps 424, 428, 432, and/or 436 as needed. In other instances, the method 400 may not include the steps 424, 428, 432, and/or 436. In other words, the method 400 may process two images to generate the lead parameter(s) and may end after the lead parameter(s) are generated.

The present disclosure encompasses embodiments of the method 400 that comprise more or fewer steps than those described above, and/or one or more steps that are different than the steps described above.

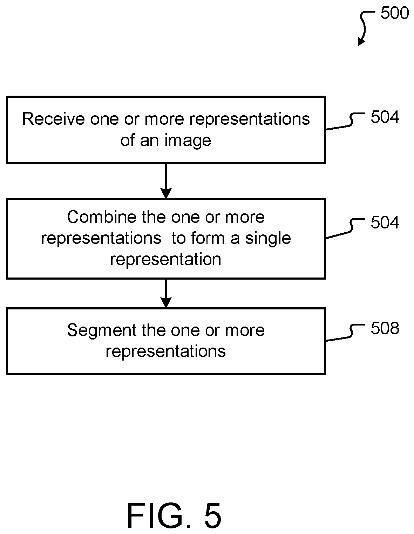

Fig. 5 depicts a non-claimed method 500 that may be used, for example, for processing one or more representations of an image.

The method 500 (and/or one or more steps thereof) may be carried out or otherwise performed, for example, by at least one processor. The at least one processor may be the same as or similar to the processor(s) 204 of the computing device 202 described above. The at least one processor may be part of a therapeutic neuromodulation system such as the therapeutic neuromodulation system 100. A processor other than any processor described herein may also be used to execute the method 500. The at least one processor may perform the method 500 by executing elements stored in a memory such as the memory 206. The elements stored in the memory and executed by the processor may cause the processor to execute one or more steps of a function as shown in method 500. One or more portions of a method 500 may be performed by the processor executing any of the contents of memory, such as an image processing 220, a segmentation 222, a registration 224, and/or a trajectory planning 228.

The method 500 comprises receiving one or more representations of an image (step 504). It will be appreciated that the first image, the second image, the third image, and any image of the methods 300 and 400 may comprise the one or more representations of the image. The image may comprise, for example, an X-ray image, a fluoroscopic image, or any other type of image. The one or more representations may comprise image data of the image. In other words, the one or more representations may be one or more derivative images of the image. For example, the one or more representations may comprise one or more optical images taken by an optical camera of an image comprising an X-ray image displayed on a display. The one or more representations may enable transferring information such as a pose of a lead relative to a target anatomical element via an optical image taken of the X-ray image as displayed on a display without setting up network servers and/or security protocols to otherwise transfer the information.

The one or more representations may be corrected for skew (e.g., the image of the X-ray may be tilted or distorted) using image processing such as the image processing 220. In other instances, an imaging device used to obtain the one or more representations may be calibrated or positioned to align with the displayed image to be captured to avoid or prevent skew.

The method 500 also comprises combining the one or more representations to form a single representation (step 508). The one or more representations may be combined using the image processing. The image processing may enable the processor to combine the one or more representations by averaging the one or more representations to form a single representation. In other embodiments, each of the one or more representations may be segmented (as described below in step 512) and each respective segmentation can be combined by the image processing by averaging the segmentations of each representation to form a single representation with the image.

The method 500 also comprises segmenting the one or more representations (step 512). A processor such as the processor 204 may use a segmentation such as the segmentation 222 to segment the one or more representations to identify one or more anatomical elements and/or one or more leads. The segmentation enables the processor to segment the image data so as to identify individual objects (e.g., lead(s)) and/or anatomical elements in the image. The segmentation may enable the processor to identify a boundary of an object or an anatomical element by using, for example, feature recognition. For example, the segmentation may enable the processor to identify a vertebra in image data. In other instances, the segmentation may enable the processor to identify a boundary of an object such as, for example, a lead such as the lead or an anatomical element by determining a difference in or contrast between colors or grayscales of image pixels.

It will be appreciated that in some embodiments, the one or more representations may be identified using any image processing or process to identify such features.

The present disclosure encompasses embodiments of the method 500 that comprise more or fewer steps than those described above, and/or one or more steps that are different than the steps described above.

As noted above, the present disclosure encompasses methods with fewer than all of the steps identified in Figs. 3, 4, and 5 (and the corresponding description of the methods 300, 400, and 500), as well as methods that include additional steps beyond those identified in Figs. 3, 4, and 5 (and the corresponding description of the methods 300, 400, and 500). The present disclosure also encompasses methods that comprise one or more steps from one method described herein, and one or more steps from another method described herein. Any correlation described herein may be or comprise a registration or any other correlation.

The foregoing is not intended to limit the disclosure to the form or forms disclosed herein. In the foregoing Detailed Description, for example, various features of the disclosure are grouped together in one or more aspects, embodiments, and/or configurations for the purpose of streamlining the disclosure. The features of the aspects, embodiments, and/or configurations of the disclosure may be combined in alternate aspects, embodiments, and/or configurations other than those discussed above. This method of disclosure is not to be interpreted as reflecting an intention that the claims require more features than are expressly recited in each claim. Rather, as the following claims reflect, inventive aspects lie in less than all features of a single foregoing disclosed aspect, embodiment, and/or configuration. Thus, the following claims are hereby incorporated into this Detailed Description, with each claim standing on its own as a separate preferred embodiment of the disclosure.

Moreover, though the foregoing has included description of one or more aspects, embodiments, and/or configurations and certain variations and modifications, other variations, combinations, and modifications are within the scope of the disclosure, e.g., as may be within the skill and knowledge of those in the art, after understanding the present disclosure. It is intended to obtain rights which include alternative aspects, embodiments, and/or configurations to the extent permitted, including alternate, interchangeable and/or equivalent structures, functions, ranges or steps to those claimed, whether or not such alternate, interchangeable and/or equivalent structures, functions, ranges or steps are disclosed herein, and without intending to publicly dedicate any patentable subject matter.

## Claims

1. A system for generating a lead parameter, comprising:
a pulse generator configured to generate an electrical signal;
an implanted lead in communication with the pulse generator and configured to transmit the electrical signal to an anatomical element;
a processor; and
a memory storing data for processing by the processor, the data, when processed, causes the processor to:
receive a first image of an anatomical region, wherein the first image depicts the anatomical element and at least a portion of an initial lead;
receive an initial lead parameter associated with the initial lead;
receive a second image of the anatomical region, wherein the second image depicts at least a portion of the anatomical element and at least a portion of the implanted lead, the second image being received at a different time than the first image; and
generate an implanted lead parameter for the implanted lead based on a combination of the initial lead parameter and a correlation of the first image with the second image.

2. The system of claim 1, wherein the anatomical region comprises a target anatomical element, and wherein the target anatomical element comprises a vertebra and the anatomical element comprises a spinal cord.

3. The system of claim 2, wherein the memory stores further data for processing by the processor that, when processed, causes the processor to:
process the first image to identify the target anatomical element and the initial lead; and
process the second image to identify the target anatomical element and the implanted lead.

4. The system of claim 2, wherein generating the lead parameter comprises:
determining a first pose of the initial lead relative to the target anatomical element in the first image and a second pose of the implanted lead relative to the target anatomical element in the second image;
determining a difference between the first pose and the second pose; and
applying the difference to the initial lead parameter to generate the implanted lead parameter.

5. The system of claim 2, wherein the memory stores further data for processing by the processor that, when processed, causes the processor to:
receive a third image of the anatomical region, the third image depicting the anatomical element and at least a portion of the implanted lead; and
generate an updated implanted lead parameter for the lead based on a combination of the implanted lead parameter and a correlation of the second image with the third image.

6. The system of claim 5, wherein generating the updated implanted lead parameter comprises:
determining a first pose of the implanted lead relative to the target anatomical element in the second image and a second pose of the implanted lead relative to the target anatomical element in the third image;
determining a difference between the first pose and the second pose; and
applying the difference to the implanted lead parameter to generate the updated implanted lead parameter.

7. The system of claim 1, wherein the first image comprises one or more representations of an X-ray image of the anatomical region and the initial lead, and wherein the memory stores further data for processing by the processor that, when processed, causes the processor to:
segmenting the one or more representations to identify the anatomical element and the initial lead.

8. The system of claim 1, wherein the first image comprises a plurality of representations of the X-ray image of the anatomical region and the initial lead, and wherein the memory stores further data for processing by the processor that, when processed, causes the processor to:
combine the plurality of representations to form a single representation.

9. The system of claim 1, wherein the memory stores further data for processing by the processor that, when processed, causes the processor to:
generate a trajectory for placing the implanted lead near the anatomical element.

10. The system of any of the preceding claims, wherein the anatomical region comprises at least one of a spinal region or a cranial region.

11. The system of claim 1, wherein the pulse generator is implantable.

12. A system for generating a lead parameter, comprising:
a processor; and
a memory storing data for processing by the processor, the data, when processed, causes the processor to:
receive a first image of an anatomical region, wherein the first image depicts an anatomical element and at least a portion of an initial lead;
process the first image to identify the anatomical element and the initial lead;
receive an initial lead parameter associated with the initial lead;
receive a second image of the anatomical region, wherein the second image depicts at least a portion of the anatomical element and at least a portion of an implanted lead, the second image taken at a different time than the first image;
process the second image to identify the anatomical element and the implanted lead; and
generate an implanted lead parameter for the lead based on a combination of the initial lead parameter, a correlation of the anatomical element of the first image with the anatomical element of the second image, and a correlation of the initial lead of the first image with the implanted lead of the second image.

13. The system of claim 12, wherein the first image comprises at least one of an optical image, a fluoroscopic image, an x-ray image, or a three-dimensional image and/or wherein the second image comprises at least one of an optical image, an x-ray image, or a three-dimensional image.

14. The system of claim 12, wherein the memory stores further data for processing by the processor that, when processed, causes the processor to:
generate a trajectory for placing the implanted lead near the anatomical element; specifically, wherein the memory stores further data for processing by the processor that, when processed, causes the processor to:
process the first image to identify the target anatomical element and the initial lead; and
process the second image to identify the target anatomical element and the implanted lead.

15. A system for generating a lead parameter, comprising:
a processor; and
a memory storing data for processing by the processor, the data, when processed, causes the processor to:
receive an optical image of an anatomical region comprising an anatomical element and an initial lead positioned near the anatomical element;
process the optical image to identify the anatomical element and the initial lead;
receive an initial lead parameter associated with the initial lead;
receive an additional image of the anatomical region and an implanted lead positioned near the anatomical element;
process the additional image to identify the anatomical element and the implanted lead;
correlate the identified anatomical element and the initial lead of the optical image and the identified anatomical element and the implanted lead of the additional image; and
generate an implanted lead parameter based on the correlation and the initial lead parameter.

## Patentansprüche

1. System zum Generieren eines Leitungsparameters, umfassend:
einen Pulsgenerator, der dazu ausgelegt ist, ein elektrisches Signal zu generieren;
eine implantierte Leitung, die in Kommunikationsverbindung mit dem Pulsgenerator steht und dazu ausgelegt ist, das elektrische Signal an ein anatomisches Element zu übertragen;
einen Prozessor; und
einen Speicher, der Daten zur Verarbeitung durch den Prozessor speichert, wobei die Daten, wenn sie verarbeitet werden, den Prozessor hierzu veranlassen:
Empfangen eines ersten Bildes einer anatomischen Region, wobei das erste Bild das anatomische Element und wenigstens einen Teil einer anfänglichen Leitung zeigt;
Empfangen eines anfänglichen Leitungsparameters, der mit der anfänglichen Leitung verknüpft ist;
Empfangen eines zweiten Bildes der anatomischen Region, wobei das zweite Bild wenigstens einen Teil des anatomischen Elements und wenigstens einen Teil der implantierten Leitung zeigt, wobei das zweite Bild zu einer anderen Zeit empfangen wird als das erste Bild; und
Generieren eines implantierten Leitungsparameters für die implantierte Leitung basierend auf einer Kombination des anfänglichen Leitungsparameters und einer Korrelation des ersten Bildes mit dem zweiten Bild.

2. System nach Anspruch 1, wobei die anatomische Region ein anatomisches Zielelement umfasst und wobei das anatomische Zielelement einen Wirbel umfasst und das anatomische Element ein Rückenmark umfasst.

3. System nach Anspruch 2, wobei der Speicher weitere Daten zur Verarbeitung durch den Prozessor speichert, die, wenn sie verarbeitet werden, den Prozessor hierzu veranlassen:
Verarbeiten des ersten Bildes, um das anatomische Zielelement und die anfängliche Leitung zu identifizieren; und
Verarbeiten des zweiten Bildes, um das anatomische Zielelement und die implantierte Leitung zu identifizieren.

4. System nach Anspruch 2, wobei das Generieren des Leitungsparameters umfasst:
Bestimmen einer ersten Pose der anfänglichen Leitung relativ zu dem anatomischen Zielelement in dem ersten Bild und einer zweiten Pose der implantierten Leitung relativ zu dem anatomischen Zielelement in dem zweiten Bild;
Bestimmen einer Differenz zwischen der ersten Pose und der zweiten Pose; und
Anwenden der Differenz auf den anfänglichen Leitungsparameter, um den implantierten Leitungsparameter zu generieren.

5. System nach Anspruch 2, wobei der Speicher weitere Daten zur Verarbeitung durch den Prozessor speichert, die, wenn sie verarbeitet werden, den Prozessor hierzu veranlassen:
Empfangen eines dritten Bildes der anatomischen Region, wobei das dritte Bild das anatomische Element und wenigstens einen Teil der implantierten Leitung zeigt; und
Generieren eines aktualisierten implantierten Leitungsparameters für die Leitung basierend auf einer Kombination des implantierten Leitungsparameters und einer Korrelation des zweiten Bildes mit dem dritten Bild.

6. System nach Anspruch 5, wobei das Generieren des aktualisierten implantierten Leitungsparameters umfasst:
Bestimmen einer ersten Pose der implantierten Leitung relativ zu dem anatomischen Zielelement in dem zweiten Bild und einer zweiten Pose der implantierten Leitung relativ zu dem anatomischen Zielelement in dem dritten Bild;
Bestimmen einer Differenz zwischen der ersten Pose und der zweiten Pose; und
Anwenden der Differenz auf den implantierten Leitungsparameter, um den aktualisierten implantierten Leitungsparameter zu generieren.

7. System nach Anspruch **1,** wobei das erste Bild eine oder mehrere Darstellungen eines Röntgenbildes der anatomischen Region und der anfänglichen Leitung umfasst und wobei der Speicher weitere Daten zur Verarbeitung durch den Prozessor speichert, die, wenn sie verarbeitet werden, den Prozessor hierzu veranlassen:
Segmentieren der ein oder mehreren Darstellungen, um das anatomische Element und die anfängliche Leitung zu identifizieren.

8. System nach Anspruch **1,** wobei das erste Bild mehrere Darstellungen des Röntgenbildes der anatomischen Region und der anfänglichen Leitung umfasst und wobei der Speicher weitere Daten zur Verarbeitung durch den Prozessor speichert, die, wenn sie verarbeitet werden, den Prozessor hierzu veranlassen:
Kombinieren der mehreren Darstellungen, um eine einzelne Darstellung zu bilden.

9. System nach Anspruch 1, wobei der Speicher weitere Daten zur Verarbeitung durch den Prozessor speichert, die, wenn sie verarbeitet werden, den Prozessor hierzu veranlassen:
Generieren einer Trajektorie zum Platzieren der implantierten Leitung in der Nähe des anatomischen Elements.

10. System nach einem der vorstehenden Ansprüche, wobei die anatomische Region wenigstens eines von einer Wirbelsäulenregion oder einer Schädelregion umfasst.

11. System nach Anspruch 1, wobei der Pulsgenerator implantierbar ist.

12. System zum Generieren eines Leitungsparameters, umfassend:
einen Prozessor; und
einen Speicher, der Daten zur Verarbeitung durch den Prozessor speichert, wobei die Daten, wenn sie verarbeitet werden, den Prozessor hierzu veranlassen:
Empfangen eines ersten Bildes einer anatomischen Region, wobei das erste Bild ein anatomisches Element und wenigstens einen Teil einer anfänglichen Leitung zeigt;
Verarbeiten des ersten Bildes, um das anatomische Element und die anfängliche Leitung zu identifizieren;
Empfangen eines anfänglichen Leitungsparameters, der mit der anfänglichen Leitung verknüpft ist;
Empfangen eines zweiten Bildes der anatomischen Region, wobei das zweite Bild wenigstens einen Teil des anatomischen Elements und wenigstens einen Teil einer implantierten Leitung zeigt, wobei das zweite Bild zu einer anderen Zeit aufgenommen wurde als das erste Bild;
Verarbeiten des zweiten Bildes, um das anatomische Element und die implantierte Leitung zu identifizieren; und
Generieren eines implantierten Leitungsparameters für die Leitung basierend auf einer Kombination des anfänglichen Leitungsparameters, einer Korrelation des anatomischen Elements des ersten Bildes mit dem anatomischen Element des zweiten Bildes und einer Korrelation der anfänglichen Leitung des ersten Bildes mit der implantierten Leitung des zweiten Bildes.

13. System nach Anspruch 12, wobei das erste Bild wenigstens eines von einem optischen Bild, einem Durchleuchtungsbild, einem Röntgenbild oder einem dreidimensionalen Bild umfasst und/oder wobei das zweite Bild wenigstens eines von einem optischen Bild, einem Röntgenbild oder einem dreidimensionalen Bild umfasst.

14. System nach Anspruch 12, wobei der Speicher weitere Daten zur Verarbeitung durch den Prozessor speichert, die, wenn sie verarbeitet werden, den Prozessor hierzu veranlassen:
Generieren einer Trajektorie zum Platzieren der implantierten Leitung in der Nähe des anatomischen Elements; wobei insbesondere der Speicher weitere Daten zur Verarbeitung durch den Prozessor speichert, die, wenn sie verarbeitet werden, den Prozessor hierzu veranlassen:
Verarbeiten des ersten Bildes, um das anatomische Zielelement und die anfängliche Leitung zu identifizieren; und
Verarbeiten des zweiten Bildes, um das anatomische Zielelement und die implantierte Leitung zu identifizieren.

15. System zum Generieren eines Leitungsparameters, umfassend:
einen Prozessor; und
einen Speicher, der Daten zur Verarbeitung durch den Prozessor speichert, wobei die Daten, wenn sie verarbeitet werden, den Prozessor hierzu veranlassen:
Empfangen eines optischen Bildes einer anatomischen Region, umfassend ein anatomisches Element und eine anfängliche Leitung, die in der Nähe des anatomischen Elements positioniert ist;
Verarbeiten des optischen Bildes, um das anatomische Element und die anfängliche Leitung zu identifizieren;
Empfangen eines anfänglichen Leitungsparameters, der mit der anfänglichen Leitung verknüpft ist;
Empfangen eines zusätzlichen Bildes der anatomischen Region und einer implantierten Leitung, die in der Nähe des anatomischen Elements positioniert ist;
Verarbeiten des zusätzlichen Bildes, um das anatomische Element und die implantierte Leitung zu identifizieren;
Korrelieren des identifizierten anatomischen Elements und der anfänglichen Leitung des optischen Bildes und des identifizierten anatomischen Elements und der implantierten Leitung des zusätzlichen Bildes; und
Generieren eines implantierten Leitungsparameters basierend auf der Korrelation und dem anfänglichen Leitungsparameter.

## Revendications

1. Système de génération d'un paramètre de dérivation, comprenant :
un générateur d'impulsions configuré pour générer un signal électrique ;
une dérivation implantée en communication avec le générateur d'impulsions et configurée pour transmettre le signal électrique à un élément anatomique ;
un processeur ; et
une mémoire stockant des données destinées à être traitées par le processeur, les données, lorsqu'elles sont traitées, amènent le processeur à :
recevoir une première image d'une région anatomique, la première image représentant l'élément anatomique et au moins une partie d'une dérivation initiale ;
recevoir un paramètre de dérivation initiale associé à la dérivation initiale ;
recevoir une deuxième image de la région anatomique, la deuxième image représentant au moins une partie de l'élément anatomique et au moins une partie de la dérivation implantée, la deuxième image étant reçue à un moment différent de la première image ; et
générer un paramètre de dérivation implantée pour la dérivation implantée sur la base d'une combinaison du paramètre de dérivation initiale et d'une corrélation entre la première image et la deuxième image.

2. Système selon la revendication 1, la région anatomique comprenant un élément anatomique cible, et l'élément anatomique cible comprenant une vertèbre et l'élément anatomique comprenant une moelle épinière.

3. Système selon la revendication 2, la mémoire stockant des données supplémentaires destinées à être traitées par le processeur qui, lorsqu'elles sont traitées, amènent le processeur à :
traiter la première image pour identifier l'élément anatomique cible et la dérivation initiale ; et
traiter la deuxième image pour identifier l'élément anatomique cible et la dérivation implantée.

4. Système selon la revendication 2, la génération du paramètre de dérivation comprenant :
la détermination d'une première position de la dérivation initiale par rapport à l'élément anatomique cible dans la première image et une deuxième position de la dérivation implantée par rapport à l'élément anatomique cible dans la deuxième image ;
la détermination d'une différence entre la première position et la deuxième position ; et
l'application de la différence au paramètre de dérivation initiale pour générer le paramètre de dérivation implantée.

5. Système selon la revendication 2, la mémoire stockant des données supplémentaires destinées à être traitées par le processeur qui, lorsqu'elles sont traitées, amènent le processeur à :
recevoir une troisième image de la région anatomique, la troisième image représentant l'élément anatomique et au moins une partie de la dérivation implantée ; et
générer un paramètre de dérivation implantée mis à jour pour la dérivation sur la base d'une combinaison du paramètre de dérivation implantée et d'une corrélation entre la deuxième image et la troisième image.

6. Système selon la revendication 5, la génération du paramètre de dérivation implantée mis à jour comprenant :
la détermination d'une première position de la dérivation implantée par rapport à l'élément anatomique cible dans la deuxième image et d'une deuxième position de la dérivation implantée par rapport à l'élément anatomique cible dans la troisième image ;
la détermination d'une différence entre la première position et la deuxième position ; et
l'application de la différence au paramètre de dérivation implantée pour générer le paramètre de dérivation implantée mis à jour.

7. Système selon la revendication 1, la première image comprenant une ou plusieurs représentations d'une image radiographique de la région anatomique et de la dérivation initiale, et la mémoire stockant des données supplémentaires destinées à être traitées par le processeur qui, lorsqu'elles sont traitées, amènent le processeur à :
segmenter la ou les représentations pour identifier l'élément anatomique et la dérivation initiale.

8. Système selon la revendication 1, la première image comprenant une pluralité de représentations de l'image radiographique de la région anatomique et de la dérivation initiale, et la mémoire stockant des données supplémentaires destinées à être traitées par le processeur qui, lorsqu'elles sont traitées, amènent le processeur à :
combiner la pluralité de représentations pour former une seule représentation.

9. Système selon la revendication 1, la mémoire stockant des données supplémentaires destinées à être traitées par le processeur qui, lorsqu'elles sont traitées, amènent le processeur à :
générer une trajectoire pour placer la dérivation implantée près de l'élément anatomique.

10. Système selon l'une quelconque des revendications précédentes, la région anatomique comprenant au moins une région spinale ou une région crânienne.

11. Système selon la revendication 1, le générateur d'impulsions étant implantable.

12. Système de génération d'un paramètre de dérivation, comprenant :
un processeur ; et
une mémoire stockant des données destinées à être traitées par le processeur, les données, lorsqu'elles sont traitées, amènent le processeur à :
recevoir une première image d'une région anatomique, la première image représentant un élément anatomique et au moins une partie d'une dérivation initiale ;
traiter la première image pour identifier l'élément anatomique et la dérivation initiale ;
recevoir un paramètre de dérivation initiale associé à la dérivation initiale ;
recevoir une deuxième image de la région anatomique, la deuxième image représentant au moins une partie de l'élément anatomique et au moins une partie d'une dérivation implantée, la deuxième image étant prise à un moment différent de la première image ;
traiter la deuxième image pour identifier l'élément anatomique et la dérivation implantée ; et
générer un paramètre de dérivation implantée pour la dérivation sur la base d'une combinaison du paramètre de dérivation initiale, d'une corrélation entre l'élément anatomique de la première image et l'élément anatomique de la deuxième image, et d'une corrélation entre la dérivation initiale de la première image et la dérivation implantée de la deuxième image.

13. Système selon la revendication 12, la première image comprenant au moins une image parmi : une image optique, une image fluoroscopique, une image radiographique ou une image tridimensionnelle et/ou la deuxième image comprenant au moins une image parmi une image optique, une image radiographique ou une image tridimensionnelle.

14. Système selon la revendication 12, la mémoire stockant des données supplémentaires destinées à être traitées par le processeur qui, lorsqu'elles sont traitées, amènent le processeur à :
générer une trajectoire pour placer la dérivation implantée près de l'élément anatomique ; spécifiquement,
la mémoire stockant des données supplémentaires destinées à être traitées par le processeur qui, lorsqu'elles sont traitées, amènent le processeur à :
traiter la première image pour identifier l'élément anatomique cible et la dérivation initiale ; et
traiter la deuxième image pour identifier l'élément anatomique cible et la dérivation implantée.

15. Système de génération d'un paramètre de dérivation, comprenant :
un processeur ; et
une mémoire stockant des données destinées à être traitées par le processeur, les données, lorsqu'elles sont traitées, amènent le processeur à :
recevoir une image optique d'une région anatomique comprenant un élément anatomique et une dérivation initiale positionnée près de l'élément anatomique ;
traiter l'image optique pour identifier l'élément anatomique et la dérivation initiale ;
recevoir un paramètre de dérivation initiale associé à la dérivation initiale ;
recevoir une image supplémentaire de la région anatomique et d'une dérivation implantée positionnée près de l'élément anatomique ;
traiter l'image supplémentaire pour identifier l'élément anatomique et la dérivation implantée ;
corréler l'élément anatomique identifié et la dérivation initiale de l'image optique et l'élément anatomique identifié et la dérivation implantée de l'image supplémentaire ; et
générer un paramètre de dérivation implantée sur la base de la corrélation et du paramètre de dérivation initiale.
